# EUROPÄISCHE PATENTSCHRIFT

(11) **EP 1 704 882 B1**
(45) Veröffentlichungstag und Bekanntmachung des Hinweises auf die Patenterteilung: **20.08.2008**
(21) Anmeldenummer: 06110076.4
(22) Anmeldetag: 17.02.2006
(51) Int. Cl.: A61M 1/34, A61M 1/16, A61M 1/36, A61M 1/32

(54) **Blutbehandlungsgerät mit Alarmvorrichtung**
Blood treatment apparatus with alarm device
Appareil de traitement de sang avec dispositif d'alarme

(30) Priorität: 23.03.2005 DE 102005013418
(43) Veröffentlichungstag der Anmeldung: 27.09.2006
(73) Patentinhaber: B. Braun Avitum AG, 49215 Glandorf (DE)
(72) Erfinder: Bock, Gerhard, 36289, Friedewald (DE); Niemetz, Günter, 34212 Melsungen (DE); Dolgos, Sándor, 2000 Szentendre (HU); Schin, Róbert, 1029 Budapest (HU)
(74) Vertreter: Selting, Günther

(56) Entgegenhaltungen:
- WO-A-99/24145
- US-A- 4 298 938
- US-A- 5 103 214
- US-A- 5 591 344
- US-A- 5 744 027

## Beschreibung

Die Erfindung betrifft ein Blutbehandlungsgerät mit einem Blutkreislauf und einem Dialysatkreislauf, Sensoren zur Überwachung von Parametern des Blutbehandlungsgerätes und/oder des Patienten und Aktoren zur Durchführung von Veränderungen in den Kreisläufen, einem Controller zur Überwachung der von den Sensoren gelieferten Signale und zur Steuerung der Aktoren, einem von dem Controller gesteuerten ersten Alarmsystem, einem den Controller überwachenden Supervisor und einem von dem Supervisor gesteuerten zweiten Alarmsystem, wobei der Controller und der Supervisor imstande sind, Alarmzustände zu detektieren.

Bei Blutbehandlungsgeräten mit extrakorporalem Blutkreislauf, wie beispielsweise Dialysegeräten, müssen zahlreiche Überwachungen durchgeführt werden, um den Patienten oder Anwender vor möglichen Gefährdungen zu schützen. Hierzu sind die Blutbehandlungsgeräte mit zahlreichen Sensoren ausgestattet, die verschiedene Parameter wie Drücke, Temperaturen, Leitfähigkeit oder Fluss überwachen und in Abhängigkeit von dem Ergebnis eine Regelung oder einen Schaltvorgang durchführen. Es ist üblich, dass bestimmte Einzelparameter bzw. Grenzwerte vom Bedienungspersonal in das Gerät eingegeben werden, und dass das Gerät die Einhaltung der Parameter und Grenzwerte während des Betriebes überwacht. Beim Vorliegen einer unakzeptablen Abweichung wird das Gerät in einen Sicherheitszustand versetzt und durch Alarmgabe wird das Pflegepersonal zu dem Gerät gerufen, um die erforderlichen Maßnahmen zu treffen, damit die Behandlung fortgesetzt werden kann. Gewöhnlich betreut eine Pflegeperson zahlreiche Patienten, so dass sie sich nicht bei jedem Alarm sofort dem betreffenden Patienten oder dem Gerät zuwenden kann. Es ist üblich, akustische Alarme im Erscheinungsbild zu differenzieren, damit die Pflegeperson bei gleichzeitigem Vorhandensein mehrerer Alarme an verschiedenen Geräten sofort erkennt, welchem Alarm bzw. welchem Gerät die höhere Priorität zukommt.

Bei Blutbehandlungsgeräten ist es bekannt, für die Steuerung und Überwachung einen Controller vorzusehen, der die Signale der Sensoren empfängt und bearbeitet und mit einem ersten Alarmsystem verbunden ist. Die hohen Sicherheitsanforderungen bei derartigen Geräten erfordern eine Überwachung des Controllers, ob dieser seine Funktion erfüllt. Diese wird von einem Supervisor vorgenommen, der ebenfalls Sensorsignale empfängt und durch Funktionsredundanz überprüft, ob der Controller in jedem Einzelfall angemessen reagiert. Der Controller und der Supervisor sind Prozessoren, die unabhängig voneinander funktionieren und sich gegenseitig überwachen. Auch der Supervisor ist mit einem eigenen Alarmsystem ausgestattet.

Dialyse-Behandlungen werden in der Regel in Dialysezentren durchgeführt. In diesen sind gewöhnlich 20 bis 50 Behandlungsplätze angeordnet, die sich auf mehrere Räume verteilen. Das Pflegepersonal führt die Behandlung nach einem ärztlichen Behandlungsplan oder direkter Weisung durch. Der Geräte betreffende Teil des Behandlungsplans wird in Form von Einstellparametern in das Gerät eingegeben. Die eingegeben Parameter werden während der Behandlung vom Gerät überwacht. Beim Vorliegen einer unakzeptablen Abweichung wird der sichere Zustand für den Patienten eingenommen und das Pflegepersonal zum Gerät gerufen, die Maßnahmen treffen, damit die Behandlung fortgesetzt werden kann. Gewöhnlich betreut eine Pflegekraft mehrere Patienten und kann sich nicht bei jedem Alarm sofort dem Patienten oder dem Gerät widmen. Damit daraus keine Gefährdung für den Patienten entsteht, werden die Alarmsignale im Erscheinungsbild der Wichtigkeit der Behandlungsabweichung variiert.

Bei einem Dialysegerät werden Konzentrate und Wasser gemischt, um den Dialysator mit Dialysierflüssigkeit zu versorgen. Das Blut fließt im Dialysator, getrennt durch eine Membran, entgegengesetzt zur Dialysierflüssigkeit. Dabei werden die Schadstoffe über die Dialysierflüssigkeit aus dem Blut entfernt. Normalerweise dauert eine Dialysebehandlung vier bis sechs Stunden. Während dieser Zeit kann es zu Zuständen kommen, die von dem Dialysegerät nicht beherrscht werden können. Solche Zustände sind zum Beispiel:
- Arterieller Druck wegen einem Verschluss in der arteriellen Leitung des extrakorporalen Kreislaufs vor der Blutpumpe zu negativ
- Venöser Druck wegen einer herausgerutschten venösen Nadel vom Patientenzugang zu niedrig
- Kein Wasser im Zulauf
- Konzentratbehälter leer
- Keine Spannungsversorgung

Zur Erkennung solcher Patienten gefährdenden Zustände sind Dialysegeräte mit zwei unabhängigen Alarmsystemen ausgerüstet, um bei einem defekten Bauteil noch sicher agieren zu können. Folgende Funktionalität ist bei jedem Alarmsystem vorhanden:
- Speicherung der Grenzwerte für die zu überwachenden Parameter
- Messwertaufnehmer für die zu überwachenden Parameter
- Messwertaufbereitung für die zu überwachenden Parameter
- Vergleich der Messwerte mit den zugehörigen Grenzwerten
- Steuerung der Aktoren, um einen behandlungssicheren Zustand einzunehmen (Blutpumpe stopp; Bypass der Dialysierflüssigkeit am Dialysator)
- Optische Anzeige des Alarmzustandes
- Akustische Anzeige des Alarmzustandes

Gewöhnlich macht der akustische Alarm das Pflegepersonal auf den unregelmäßigen Zustand des Gerätes aufmerksam. Störend ist, wenn ein Alarmzustand von dem ersten Alarmsystem bereits gemeldet wurde und das zweite Alarmsystem den gleichen Zustand ebenfalls meldet, da damit eine höhere Aufmerksamkeit dem Pflegepersonal suggeriert wird, obwohl diese nicht nötig ist.

In DE 199 01 288 A1 ist eine Vorrichtung zur Überwachung von Lautsprechern beschrieben, mit der die Funktionsfähigkeit einzelner Lautsprecher durch Aufnahme eines von dem Lautsprecher abgestrahlten akustischen Signals überwacht werden kann. Damit kann ein Ausfall eines Lautsprechers erkannt werden.

In W99/24145 wird ein Computer überwachtes Dialysebehandlungsgerät offenbart, bei dem zwei Prozessoren Sensoren überwachen und Aktoren ansteuern können. Sowohl der Hauptprozessor als auch der Sicherheitsprozessor können ein erstes Alarmsystem ansteuern. Ein zweites Alarmsystem wird nur von dem Sicherheitsprozessor angesteuert.

In US5103214 wird ein Alarmsystem mit zwei Alarmgebern vorgestellt, bei dem der zweite Alarmgeber misst, ob der erste Alarmgeber ein Signal von sich gibt. Ein Prozessor wertet aus, ob eine Alarmbedingung vorliegt und steuert den ersten Alarmgeber an. Falls der zweite Alarmgeber kein Signal des ersten Alarmgebers misst, löst der Prozessor den zweiten Alarmgeber aus.

Der Erfindung liegt die Aufgabe zugrunde, ein Blutbehandlungsgerät mit Controller und Supervisor, die jeweils über ein eigenes Alarmsystem verfügen, zu schaffen, bei dem im Störungsfall eine sichere und geordnete Alarmerzeugung unter Vermeidung von Doppelalarm erfolgt.

Das erfindungsgemäße Blutbehandlungsgerät ist durch den Patentanspruch 1 bezeichnet. Es zeichnet sich dadurch aus, dass bei Detektion eines Alarmzustandes von Controller und/oder Supervisor nur das erste Alarmsystem, also dasjenige des Controllers, aktiviert wird, und dass eine Funktionsüberwachungsvorrichtung vorgesehen ist, die im Falle des Versagens des ersten Alarmsystems über den Supervisor das zweite Alarmsystem aktiviert.

Die Erfindung sieht vor, dass die akustische Signalisierung über das erste Alarmsystem durchgeführt wird und der Supervisor die ordnungsgemäße Funktion dieses Alarmsystems überwacht. Es wird also sichergestellt, dass nur eines der Alarmsysteme aktiviert wird, und dass nicht beide Alarmsysteme miteinander in Konkurrenz treten. Dadurch wird eine Verwirrung des Pflegepersonals vermieden. Dabei wird das Pflegepersonal bei der Behebung von Alarmzuständen stressfrei unterstützt. Unnötige Handhabungen werden vermieden. Die Erfindung eignet sich insbesondere für solche Blutbehandlungsgeräte, die in einem Dialysezentrum aufgestellt sind, kann jedoch auch bei Einzelgeräten Anwendung finden.

Es besteht die Möglichkeit, dass an demselben Blutbehandlungsgerät mehrere Alarmzustände gleichzeitig gemeldet werden. In solchen Fällen gilt es, den gefährlicheren Alarmzustand zuerst zu melden und zu beheben. In vorteilhafter Weiterbildung der Erfindung ist vorgesehen, dass in Abhängigkeit von den Signalen der Sensoren die Alarmzustände in unterschiedliche Alarmstufen unterteilt sind, wobei bei gleichzeitigem Vorhandensein mehrerer Alarmzustände derjenige mit der höchsten Alarmstufe aktiviert wird. Dies bedeutet, dass der Alarm mit der höchsten Alarmstufe das Alarmsystem des Controllers aktiviert und dieser Alarm gleichzeitig an einem Bediengerät, das die Benutzerschnittstelle darstellt, angezeigt wird, eventuell zusammen mit Gegenmaßnahmen, die durch das Personal einzuleiten sind. Sobald der Alarmzustand mit der höchsten Alarmstufe behoben wurde, wird der nächstrangige Alarmzustand angezeigt.

Hierdurch wird ein geordnetes Abarbeiten der einzelnen Alarmzustände ermöglicht.

Eine bevorzugte Ausführungsform der Erfindung sieht vor, dass im Controller und im Supervisor jeweils eine Alarmtabelle gebildet wird, die diejenigen Alarmzustände enthält, die mit den Sensoren des Controllers bzw. Supervisors detektiert wurden. Zusätzlich kann ein Abgleich der beiden Alarmtabellen durchgeführt werden. Der Abgleich bedeutet, dass eine Alarmtabelle, die einen Alarmzustand nicht enthält, ergänzt wird, wobei dieser Alarmzustand in der Alarmtabelle hinzugefügt wird. In jedem Fall wird sichergestellt, dass beide Alarmtabellen gleich sind, so dass der Supervisor auch die korrekte Alarmerzeugung durch den Controller überwachen kann.

Die Alarmstufen können unterschiedliche Klangbilder in Frequenz und/oder Tonrhythmus oder Klangbild aufweisen.

Zweckmäßigerweise ist eine Alarmquittierungstaste vorgesehen, bei deren Betätigung die Alarmerzeugung für eine vorgegebene Zeit ausgesetzt wird. Dadurch ist sichergestellt, dass die Bedienungsperson zunächst einmal die Alarmerzeugung abschalten kann, bevor die Fehlerbehebung bzw. die erforderlichen Maßnahmen am Patienten erfolgen. Auf diese Weise wird zunächst einmal die erforderliche Ruhe hergestellt. Nach Ablauf der vorgesehenen Zeit wird allerdings der Alarm von Neuem erzeugt, falls er bis dahin nicht behoben wurde.

Im Folgenden wird ein Ausführungsbeispiel der Erfindung unter Bezugnahme auf die Zeichnungen näher erläutert.

Es zeigen:
- Figur 1: eine Gesamtdarstellung des Blutbehandlungsgerätes,

- Figur 2: eine Darstellung des Bediengerätes,
- Figur 3: eine schematische Darstellung des Steuer- und Überwachungssystems,
- Figur 4: eine schematische Darstellung der Funktionsüberwachungsvorrichtung für das erste Alarmsystem, und
- Figuren 5a und 5b: ein Flussdiagramm der Funktion von Controller und Supervisor.

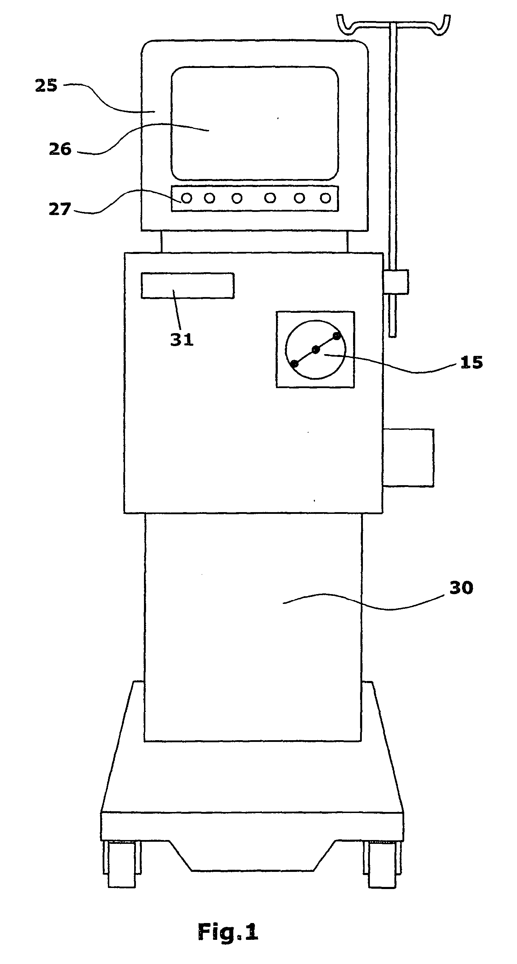

Das in Figur 1 dargestellte Blutbehandlungsgerät ist auf Rollen fahrbar. Es weist einen Sockel 30 auf. An der Frontseite ist die Blutpumpe 15 angebracht, bei der es sich um eine Schlauchpumpe handelt. Am oberen Ende des Gehäuses befindet sich eine Spritzenpumpe 31. Auf dem Gehäuse ist das Bediengerät 25 montiert, das einen Bildschirm 26 und einen Bedienteil 27 aufweist. Die Aufbereitung des Dialysates erfolgt im Inneren des Gehäuses. Das Gerät wird mit Schläuchen an den Blutkreislauf des Patienten angeschlossen.

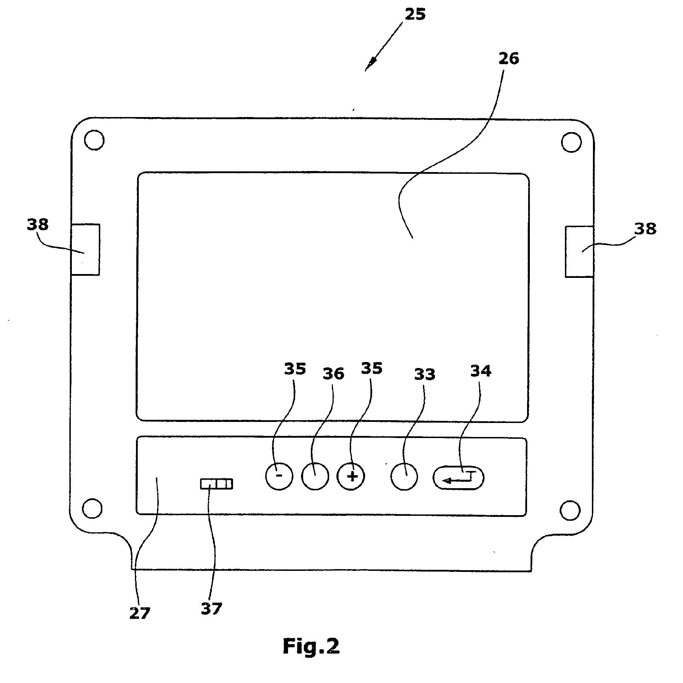

Figur 2 zeigt das Bediengerät 25 mit dem Bildschirm 26, der als Touchscreen ausgebildet ist, und dem darunter angeordneten Tastenfeld 27. Das Tastenfeld 27 enthält eine Alarmquittierungstaste 33, eine Entertaste 34 sowie Eingabetasten 35 zum Erhöhen oder Erniedrigen von Eingabewerten für die Blutpumpe sowie eine Start/Stopptaste 36 für die Blutpumpe und eine Batteriezustandsleuchte 37. Seitlich an dem Bediengerät 25 sind Signalleuchten 38 angebracht. Die Signalleuchten 38 leuchten grün und zeigen damit den alarmfreien Zustand an. Sie leuchten rot und zeigen damit den Alarmzustand an.

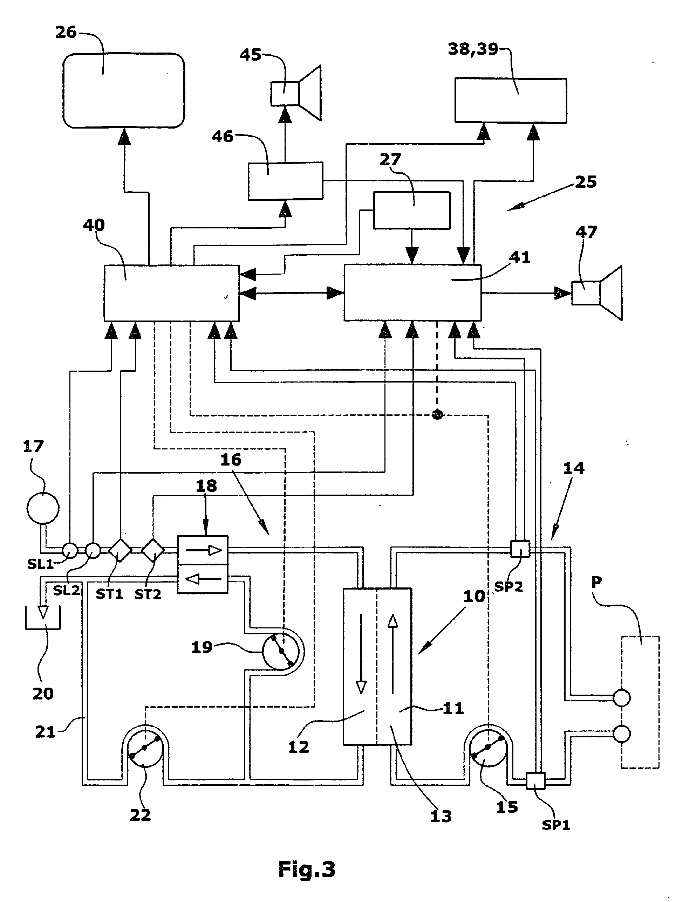

In Figur 3 ist das Kreislaufsystem der Dialysemaschine schematisch dargestellt. Die Dialysemaschine weist einen Dialysator 10 auf mit einer ersten Kammer 11 und einer zweiten Kammer 12, die durch eine Dialysemembran 13 voneinander getrennt sind. Die erste Kammer 11 ist eine Blutkammer, die Bestandteil eines Blutkreislaufs 14 ist. Der Blutkreislauf führt von dem Körper eines Patienten P über eine Blutpumpe 15 durch die Kammer 11 hindurch zurück zum Patienten. Die zweite Kammer 12 ist Bestandteil eines Dialysatkreislaufs 16, der durch die zweite Kammer 12 führt. Der Dialysatkreislauf 16 führt von einer Dialysatquelle 17, welche Dialysierflüssigkeit liefert, durch eine Bilanziereinrichtung 18 zu der zweiten Kammer 12 und von dort über eine Dialysatpumpe 19 zu der Bilanziereinrichtung 18 und dann in einen Ablauf 20. Die Bilanziereinrichtung 18 bewirkt, dass die abgeführte Flüssigkeitsmenge gleich der zugeführten Flüssigkeitsmenge ist. Hinter dem Dialysator 10 zweigt eine Bypassleitung 21 ab, die eine weitere Pumpe 22 enthält und direkt in den Ablauf 20 führt. Die Bypassleitung 21 ist eine Ultrafiltrationsleitung. Durch Einstellung des Fördervolumens der Pumpe 22 kann das Ultrafiltrationsvolumen bestimmt werden, also dasjenige Volumen, das durch die Membran 13 des Dialysators hindurch zusätzlich in den Dialysatkreislauf 16 übertragen wird.

Alle Pumpen 15, 19 und 22 sind volumetrische Pumpen oder Verdrängerpumpen, bei denen das Fördervolumen zur Pumpengeschwindigkeit proportional ist. Die Pumpengeschwindigkeiten werden von dem Steuerteil des Bediengerätes 25 gesteuert, das die Anzeigevorrichtung in Form eines Bildschirms 26 aufweist, und dem Bedienteil 27 mit mehreren Tasten. Der Bildschirm 26 bildet die Benutzerschnittstelle zum Einstellen und Ändern von Maschinenparametern und zur Information des Benutzers über den Betrieb der Dialysemaschine.

Das Bediengerät 25 enthält einen ersten Prozessor, der als Controller 40 bezeichnet wird und einen zweiten Prozessor, der als Supervisor 41 bezeichnet wird. Der Controller 40 ist mit dem Bildschirm 26 verbunden. Er dient zur internen Steuerung des Blutbehandlungsgerätes und der verschiedenen darin enthaltenen Komponenten. In dem Blutbehandlungsgerät sind zahlreiche Sensoren vorhanden, von denen in der Zeichnung nur einige Exemplare dargestellt sind. Hierzu gehören Leitfähigkeitssensoren SL1 und SL2 und Temperatursensoren ST1,ST2 in der Leitung zwischen der Dialysatquelle 17 und der Bilanziereinrichtung 18, sowie ein Drucksensor SP1 der arteriellen Blutleitung und ein Drucksensor SP2 in der venösen Blutleitung des Blutkreislaufs 14. Die Anzahl der insgesamt vorhandenen Sensoren, die verschiedene Funktionen und Parameter überwachen, einschließlich der Zubereitung des Dialysats in der Dialysatquelle 17, beträgt generell 30-40. Die Signale der Sensoren werden an den Controller 40 geliefert. Einige dieser Signale werden zusätzlich an den Supervisor 41 geliefert. Von den beiden Leitfähigkeitssensoren SL1 und SL2 und den beiden Temperatursensoren ST1 und ST2 ist jeweils einer (SL1,ST1) mit dem Controller 40 verbunden und der andere (SL2,ST2) mit dem Supervisor 41. Diese Sensoren sind doppelt vorhanden, damit ein Ausfall eines dieser Sensoren erkannt wird. Andere Sensoren liefern ihre Ausgangssignale sowohl an den Controller 40 als auch an den Supervisor 41.

Der Controller 40 bildet eine selbständig funktionsfähige Steuereinheit zur Steuerung sämtlicher Funktionen des Gerätes. Er steuert die Pumpen 15,19 und 22 und regelt die gesamten Abläufe in dem Gerät entsprechend den vom Benutzer vorgegebenen Parametern und der jeweils eingestellten Betriebsart.

Der Controller 40 ist mit einem eigenen Alarmsystem 45 versehen, das mit einer Funktionsüberwachungsvorrichtung 46 ausgestattet ist. Das Ausgangssignal der Funktionsüberwachungsvorrichtung 46 wird an den Supervisor 41 geliefert. Dieser ist mit einem eigenen Alarmsystem 47 ausgestattet. Die Alarmsysteme 45 und 47 weisen jeweils einen akustischen Alarmgeber auf, der Alarme in unterschiedlichen Alarmstufen erzeugen kann. Die Alarmstufen unterscheiden sich durch Frequenz bzw. Klangfarbe oder Tonrhythmus der Schallsignale.

Die vom Benutzer eingegebenen Behandlungsparameter, beispielsweise Sollwerte oder Grenzwerte, werden sowohl im Controller als auch im Supervisor gespeichert. Während der Behandlung des Patienten erfolgt eine zweikanalige Überwachung, nämlich durch den Controller und den Supervisor. Im Controller 40 und im Supervisor 41 werden die von den einzelnen Sensoren gelieferten Werte mit den zugehörigen Grenzwerten verglichen. Liegt ein Sensorwert außerhalb der vorgegebenen Grenzen, wird ein für den Patienten sicherer Zustand eingestellt. Dies geschieht entsprechend dem Ort des Sensors. Liegt z.B. eine Grenzwertüberschreitung bei der Aufbereitung der Dialysierflüssigkeit vor, wird die Dialysierflüssigkeit durch einen (nicht dargestellten) Bypass am Dialysator vorbeigeleitet. Dies geschieht, indem entsprechende Ventile umgeschaltet werden. Im Blutkreislauf werden die Blutpumpe 15 und eine Spritzenpumpe 31 gestoppt und entsprechende (nicht dargestellte) Schlauchklemmen geschlossen. Gleichzeitig werden die Signalleuchten 38 auf Rot geschaltet.

Anschließend führen Controller 40 und Supervisor 41 ein Update der Alarmtabelle durch, wobei der betreffende Zustand vermerkt und gespeichert wird. In der Alarmtabelle wird auch die Alarmstufe des jeweiligen Alarms vermerkt. Die Alarmstufen sind aufgeteilt in: hohe, mittlere und niedrige Priorität.

In der nachfolgenden Tabelle 1 sind die möglichen Folgen angegeben, die eintreten können, wenn auf einen Alarm nicht reagiert wird. Daraus ergeben sich die verschiedenen Prioritäten oder Alarmstufen.

**Tabelle 1**

| Mögliche Folge der Unterlassung, auf den Alarm zu reagieren | Beginn der möglichen Schädigung | | |
|---|---|---|---|
| | unmittelbar | prompt | verzögert |
| Tod oder irreversible Verletzung | Hohe Priorität | Hohe Priorität | Mittlere Priorität |
| Reversible Verletzung | Hohe Priorität | Mittlere Priorität | Niedrige Priorität |
| Geringfügige Verletzung | Mittlere Priorität | Mittlere Priorität | Niedrige Priorität |

Die akustische Alarmierung erfolgt zunächst entsprechend der höchsten Alarmstufe, die in der Alarmtabelle eingetragen ist. Die Signalgebung über Lautsprecher geschieht im Impulsbetrieb, wobei einzelne Töne durch Pausen getrennt sind. Dabei können entsprechend der jeweiligen Alarmstufe unterschiedliche Signalmuster erzeugt werden.

Wenn der Controller 40 eine Alarmsituation festgestellt hat, wird diese an den Supervisor mitgeteilt. Der Supervisor prüft anhand einer Strommessung die Ausführung des Alarms.

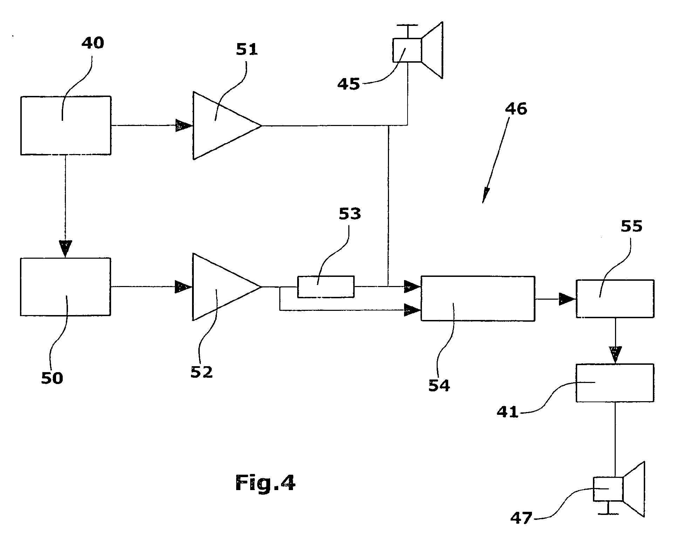

In Figur 4 ist die Funktionsüberwachungsvorrichtung 46 des Alarmsystems 45 dargestellt. Die vom Controller 40 erzeugten Alarmsignale werden einem Frequenzgenerator 50 zugeführt und ferner zur Steuerung des Alarmsystems 45 einem Verstärker 51. Der Lautsprecherstrom des Alarmsystems 45 wird als Spannungsabfall über einen Widerstand 53 gemessen und von einer Messschaltung 54 erkannt. Die Messschaltung 54 misst den Lautsprecherstrom und die Frequenz der Lautsprechersignale. Beides wird über einen Analog/Digital-Wandler 55 in Digitalsignale umgewandelt und dem Supervisor 41 zugeführt. Dieser überwacht somit die Signalerzeugung und auch die Korrektheit des betreffenden Alarmsignals. Ist das Alarmsignal korrekt, wird das zweite Alarmsystem 47 vom Supervisor nicht betätigt. Der Supervisor 41 betätigt das Alarmsystem 47 nur dann, wenn das erste Alarmsystem 45 trotz des Vorhandenseins eines Alarmzustands nicht aktiviert wurde. Der Supervisor überwacht also u.a. auch die Funktion des Lautsprechers im Alarmsystem 45. Falls der Lautsprecher defekt ist, wird dies durch das Ausbleiben des Lautsprecherstroms erkannt.

Befindet sich eine Pflegeperson an dem Blutbehandlungsgerät, um die Alarmursache zu beheben, kann die Pflegeperson die Alarmquittierungstaste 33 (Figur 2) drücken, um das Alarmsystem für eine vorbestimmte Zeit still zu setzen. Nach behobener Alarmursache betätigt die Pflegeperson wiederum die Alarmquittierungstaste. Daraufhin wird vom Controller 40 oder vom Supervisor 41 die rote Signalleuchte ausgeschaltet und die Aktoren für die Befehle des Controllers werden wieder freigegeben, so dass die Behandlung fortgeführt wird.

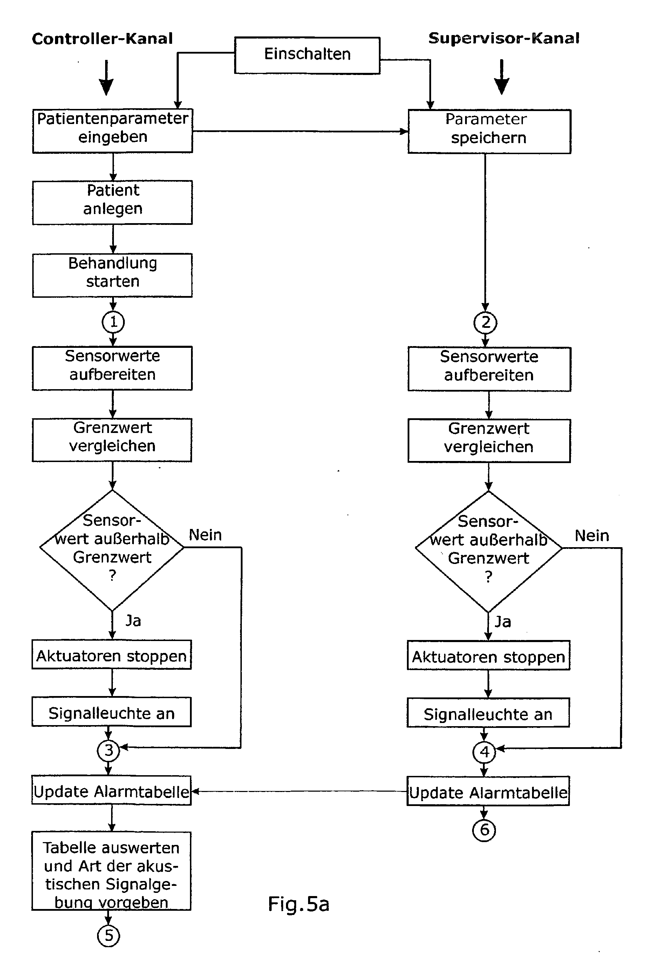

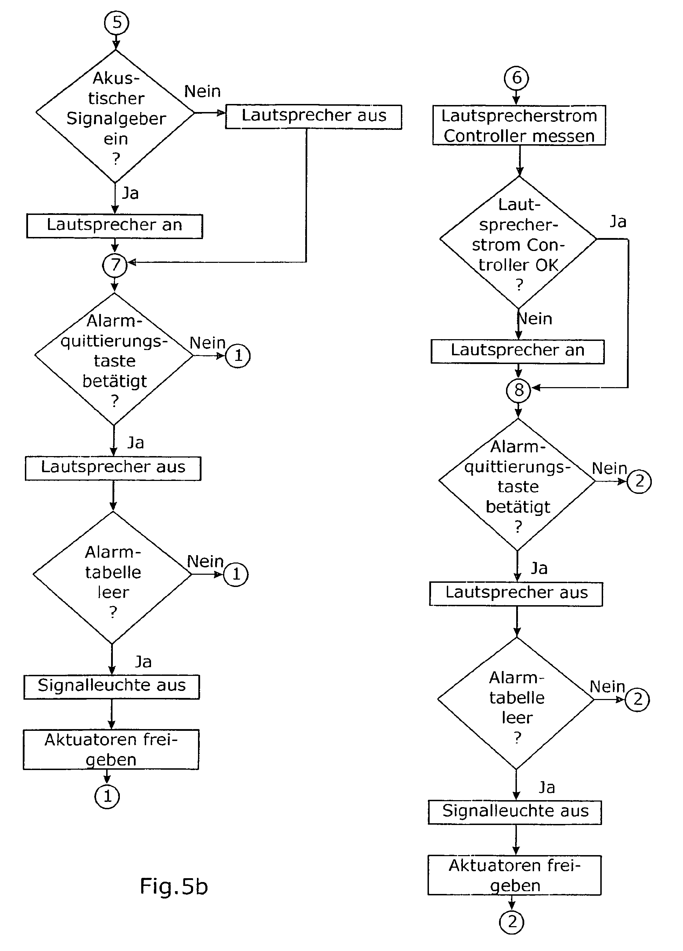

Die Figuren 5a und 5b zeigen ein Flussdiagramm der Abläufe in dem Controller-Kanal des Controllers 40 und dem Supervisor-Kanal des Supervisors 41.

Der Schritt "Sensorwerte aufbereiten" umfasst das Abtasten sämtlicher Sensoren. Die Abtastung erfolgt zyklisch alle 200 ms. Wie sich aus Figur 5a ergibt, werden die Funktionen generell in beiden Kanälen parallel durchgeführt. In beiden Kanälen wird eine Alarmtabelle der erkannten Alarmzustände geführt. Beide Alarmtabellen werden miteinander verglichen. Der Supervisor überträgt seine Alarmtabelle zum Controller, der diese Alarmzustände in seine Alarmtabelle einträgt. Es ist auch möglich, dass die Alarmtabelle des Controllers zum Supervisor übertragen wird.

Wie sich aus Figur 5b ergibt, wird im normalen Alarmfall nur der Lautsprecher des vom Controller gesteuerten Alarmsystems 45 betätigt, während das zweite Alarmsystem 47 inaktiv bleibt. Das zweite Alarmsystem 47 des Supervisors wird nur dann aktiviert, wenn der Lautsprecherstrom des Alarmsystems des Controllers ausbleibt oder nicht korrekt ist. Nach Betätigung der Alarmquittierungstaste 33 wird der Alarm ausgesetzt, d.h. der Lautsprecher ausgeschaltet. Dann wird geprüft, ob die Alarmtabelle leer ist. Wenn kein weiterer Alarm ansteht, wird die rote Signalleuchte gelöscht und die grüne Signalleuchte eingeschaltet und das Gerät kehrt zu den Zuständen 1 und 2 in Figur 5a zurück.

## Patentansprüche

1. Blutbehandlungsgerät mit einem Blutkreislauf (14) und einem Dialysatkreislauf (16), Sensoren zur Überwachung von Parametern und Aktoren zur Durchführung von Veränderungen in den Kreisläufen, einem Controller (40) zur Überwachung der von den Sensoren gelieferten Signale und zur Steuerung der Aktoren, einem von dem Controller (40) gesteuerten ersten Alarmsystem (45), einem den Controller (40) überwachenden Supervisor (41) und einem von dem Supervisor gesteuerten zweiten Alarmsystem (47), wobei der Controller (40) und der Supervisor (41) imstande sind, Alarmzustände zu detektieren, wobei
bei Detektion eines Alarmzustandes von Controller (40) und/oder Supervisor (41) das erste Alarmsystem (45) aktiviert wird, und **dadurch gekennzeichnet, dass** eine Funktionsüberwachungsvorrichtung (46) vorgesehen ist, die im Falle des Versagens des ersten Alarmsystems (45) über den Supervisor (41) das zweite Alarmsystem (47) aktiviert.

2. Blutbehandlungsgerät nach Anspruch 1, **dadurch gekennzeichnet, dass** in Abhängigkeit von den Signalen der Sensoren die Alarmzustände in unterschiedliche Alarmstufen unterteilt sind, wobei bei gleichzeitigem Vorhandensein mehrerer Alarmzustände derjenige mit der höchsten Alarmstufe aktiviert wird.

3. Blutbehandlungsgerät nach Anspruch 1 oder 2, **dadurch gekennzeichnet, dass** im Controller (40) und im Supervisor (41) jeweils eine Alarmtabelle gebildet wird, die diejenigen Alarmzustände enthält, die mit den Sensoren des Controllers (40) bzw. Supervisors (41) detektiert wurden.

4. Blutbehandlungsgerät nach Anspruch 2, **dadurch gekennzeichnet, dass** die Alarmstufen unterschiedliche Klangbilder in Frequenz und/oder Tonrhythmus aufweisen.

5. Blutbehandlungsgerät nach einem der Ansprüche 1 bis 4, **dadurch gekennzeichnet, dass** eine Alarmquittierungstaste (33) vorgesehen ist, bei deren Betätigung der Alarm für eine vorgegebene Zeit ausgesetzt wird.

6. Blutbehandlungsgerät nach Anspruch 3, **dadurch gekennzeichnet, dass** der Alarm in Abhängigkeit von den in der Alarmtabelle enthaltenen Alarmzuständen erzeugt wird.

7. Blutbehandlungsgerät nach einem der Ansprüche 1 bis 6, **dadurch gekennzeichnet, dass** eine Abtastung der Sensoren in festgelegten Zeitabständen erfolgt.

## Claims

1. A blood treatment apparatus comprising a blood circulation (14) and a dialysate circulation (16), sensors for monitoring parameters and actors for effecting changes in the circulations, a controller (40) for monitoring the signals supplied from the sensors and for controlling the actors, a first alarm system (45) controlled by the controller (40), a supervisor (41) monitoring the controller (40) and a second alarm system (47) controlled by the supervisor, the controller (40) and the supervisor (41) being configured to detect alarm conditions, wherein, upon detection of an alarm condition by the controller (41) and/or the supervisor (41), the first alarm system (45) is activated, and **characterized in that** a function monitoring device (46) is provided which, in case of a failure of the first alarm system (45), activates the second alarm system (47) through the supervisor (41).

2. The blood treatment apparatus of claim 1, wherein the alarm conditions are divided into different alarm stages depending on the signals from the sensors, the alarm condition of the highest alarm stage being activated if several alarm conditions should be present at the same time.

3. The blood treatment apparatus of claim 1 or 2, wherein an alarm table is established in the controller (40) and in the supervisor (41), respectively, which includes the alarm conditions detected by the sensors of the controller (41) or the supervisor (41), respectively.

4. The blood treatment apparatus of claim 2, wherein the alarm stages have sounds differing in frequency and/or tonal rhythm.

5. The blood treatment apparatus of one of claims 1 to 4, wherein an alarm acknowledgement key (33) is provided whose operation halts the alarm for a predetermined period of time.

6. The blood treatment apparatus 3, wherein the alarm is issued depending on the alarm conditions contained in the alarm table.

7. The blood treatment apparatus of one of claims 1 to 6, wherein the sensors are sampled in fixed time intervals.

## Revendications

1. Appareil pour le traitement du sang comportant un circuit pour le sang (14) et un circuit pour le dialysat (16), des capteurs pour surveiller les paramètres et des actionneurs pour effectuer les modifications dans les circuits, un contrôleur (40) pour surveiller les signaux fournis par les capteurs et pour commander les actionneurs, un premier système d'alarme (45) commandé par le contrôleur (40), un superviseur (41) surveillant le contrôleur (40) et un deuxième système d'alarme (47) commandé par le superviseur, sachant que le contrôleur (40) et le superviseur (41) sont en mesure de détecter des états d'alarme, le premier système d'alarme (45) est activé en cas de détection d'un état d'alarme par le contrôleur (40) et/ou le superviseur (41) **caractérisé en ce qu'**on prévoit un dispositif de surveillance du fonctionnement (46) qui active le deuxième système d'alarme (47), par l'intermédiaire du superviseur (41) en cas de défaillance du premier système d'alarme (45).

2. Appareil pour le traitement du sang selon la revendication 1, **caractérisé en ce que** les états d'alarme sont divisés en différents niveaux d'alarme en fonction des signaux émis par les capteurs, sachant qu'en présence de plusieurs états d'alarme simultanés, c'est celui qui a le plus haut niveau d'alarme qui est activé.

3. Appareil pour le traitement du sang selon la revendication 1 ou la revendication 2, **caractérisé en ce qu'**un tableau des alarmes contenant les états d'alarme qui ont été détectées avec les capteurs du contrôleur (40) respectivement du superviseur (41), est créé dans le contrôleur (40) et dans le superviseur (41).

4. Appareil pour le traitement du sang selon la revendication 2, **caractérisé en ce que** les niveaux d'alarme présentent des schémas sonores différents en fréquence et/ou en rythme sonore.

5. Appareil pour le traitement du sang selon l'une quelconque des revendications 1 à 4, **caractérisé en ce qu'**on prévoit une touche d'acquittement (33) de l'alarme qui, lorsqu'on l'actionne, met l'alarme hors service pendant un laps de temps prédéfini.

6. Appareil pour le traitement du sang selon la revendication 3, **caractérisé en ce que** l'alarme est produite en fonction des états d'alarme contenus dans le tableau des alarmes.

7. Appareil pour le traitement du sang selon l'une quelconque des revendications 1 à 6, **caractérisé en ce que** les capteurs sont balayés à des intervalles de temps fixes.
